**Europäisches Patentamt**

(19) **European Patent Office**

**Office européen des brevets**

(11) Numéro de publication: **0 140 764**
**B1**

(12) **FASCICULE DE BREVET EUROPÉEN**

(45) Date de publication du fascicule du brevet:
**21.03.90**

(21) Numéro de dépôt: **84401974.5**

(22) Date de dépôt: **03.10.84**

(51) Int. Cl.⁵: **C 12 Q   1/68**

(54) **Procédé et réactifs pour la détection d'un acide nucléique normalement monocaténaire, notamment d'un ARN, dans une matière biologique, notamment dans une préparation de leucocytes.**

(30) Priorité: **03.10.83 FR 8315726**

(43) Date de publication de la demande:
**08.05.85 Bulletin 85/19**

(45) Mention de la délivrance du brevet:
**21.03.90 Bulletin 90/12**

(84) Etats contractants désignés:
**AT BE CH DE GB IT LI LU NL SE**

(56) Documents cité:
**EP-A-0 070 687**
**WO-A-83/02286**
**FR-A-2 152 477**
**FR-A-2 505 845**
**GB-A-2 019 408**
**US-A-4 038 143**

(73) Titulaire: **INSTITUT PASTEUR**
**25-28, rue du Docteur Roux**
**F-75724 Paris Cédex 15 (FR)**

(72) Inventeur: **Crepin, Michel**
**25, rue des Cordelières**
**F-75013 Paris (FR)**

(74) Mandataire: **Gutmann, Ernest**
**S.C. Ernest Gutmann - Yves Plasseraud 67, boulevard Haussmann**
**F-75008 Paris (FR)**

EP 0 140 764 B1

## Description

L'invention est relative à un procédé et à des réactifs pour la détection d'une anomalie génétique dans une matière biologique complexe, par exemple un tissu ou, de préférence, une préparation de cellules du sang, telles que des leucocytes. Elle concerne plus particulièrement l'application de ce procédé à des détections in vitro d'anomalies génétiques mettant notamment en jeu des gènes défectifs des oncogènes ou des gènes exogènes susceptibles de s'incorporer aux tissus, leucocytes ou autres cellules de l'organisme et de s'y exprimer.

Sous l'expression "anomalie génétique", on entend soit toute anomalie génétique se manifestant par une expression et impliquant par conséquent l'apparition précoce d'un ARN messager (mARN), normalement non synthétisé par l'hôte, soit au contraire l'absence ou la disparition d'un mARN normalement synthétisé par l'hôte. Dans le premier cas (apparition d'un ARN messager normalement non présent chez l'hôte), le mARN peut refléter soit une altération du patrimoine génétique de l'hôte, soit une induction de l'expression de gènes présents dans l'organisme, mais normalement non exprimés, soit une invasion ou une infiltration de l'organisme ou l'hôte dont provient la matière par des constituants étrangers, porteurs de gènes susceptibles d'être eux-mêmes exprimés dans cet organisme.

On citera à titre d'exemples d'anomalies génétiques, le oncogènes souvent remaniés, qui s'expriment dans les tumeurs correspondantes. Un certain nombre de gènes impliqués dans des oncogénèses ont déjà été décrits dans la littérature (Myb, Myc, Erb ...). Par exemple, le gène Myc est activé dans les myélocytomatoses.

Parmi ces produits peuvent encore également se trouver les produits d'expression d'agents pathogènes ayant envahi l'organisme à un stade antérieur.

On appréciera que le traitement des affections qui peuvent être liées à ces anomalies génétiques sera d'autant plus efficace qu'elles auront été dépistées plus tôt.

L'article "Quick-Blot": Selective mRNA or DNA immobilization from Whole Cells", de Bresser et al. (DNA volume 2, Number 3, 1983, pages 243 à 254) décrit un procédé d'immobilisation d'ADN ou d'ARN messager à des fins d'hybridation par une sonde radioactive, lequel procédé comprend les étapes suivantes:

– on lyse les cellules et on traite les fractions sous-cellulaires obtenues à l'aide d'une protéase et d'iodure de sodium,
– on filtre les solutions obtenues pour déposer l'ADN (ou l'ARN),
– on fait tremper les filtres dans de l'eau puis dans un mélange d'eau et d'éthanol pour éliminer l'excès d'iodure de sodium,
– on fait tremper les filtres dans une solution d'anhydride acétique pour acétyler les protéines,
– on agite les filtres sur lesquels est déposé l'ADN (ou l'ARN) en présence de dodécylsulfate de sodium,
– on met les filtres sur lesquels se trouve l'ADN ou (l'ARN) en contact avec une solution contenant du dodécyl sulfate de sodium et une sonde radioactive.

L'invention vise par conséquent à promouvoir un procédé de détection qui puisse rendre compte des stades les plus précoces du développement de l'affection recherchée. Elle s'est par conséquent particulièrement intéressée aux premières séquences des séries d'événements qui, au plan biologique, aboutissent aux produits d'expression caractéristiques de l'affection ou de l'anomalie étudiée. L'un de ces premiers événements consiste dans la transcription en mARN de l'anomalie génétique qui en est la cause.

Dans son aspect le plus général, l'invention a également pour but de fournir un procédé permettant d'opérer des détections de la présence ou non d'un ARN au sein d'un échantillon biologique, qui soit à la fois plus sensible et plus simple à mettre en oeuvre que les procédés classiques de détection d'un ARN particulier. En particulier, l'invention vise à remédier aux difficultés qu'entraîne la mise en oeuvre des procédés classiques, ceux-ci impliquant en général des purifications poussées des ARN avant que soit envisagée la mise en contact de ces derniers avec une sonde susceptible d'hybrider avec l'ARN particulier recherché. Plus particulièrement, l'invention a donc aussi pour but de réduire dans une importante proportion les traitements préliminaires nécessaires à l'obtention d'une préparation d'ARN dans un état convenable pour que l'étape de détection, notamment par hybridation avec une sonde marquée, puisse effectivement être mise en oeuvre. Elle a par conséquent encore pour but de réduire les pertes d'ARN à détecter qu'entraînent les procédures usuelles de purification.

Dans son aspect le plus large, le procédé selon l'invention de détection in vitro d'un acide nucléique normalement monocaténaire, notamment d'un ARN déterminé, dans une matière biologique le contenant et contenant des cellules, comprenant les étapes suivantes:

– le traitement de la matière biologique par la mise en contact de ladite matière avec un détergent non ionique pour solubiliser les membranes des cellules contenues dans la matière biologique et libérer une partie des constituants cellulaires internes y inclus les ADN, ARN, et plus particulièrement les ARN messagers,
– la dissociation, avant l'hybridation des complexes protéine-ARN monocaténaire et le démasquage de l'ARN monocaténaire à l'aide d'une solution d'un sel ionique, de molarité de 5 à 13 M, ce traitement ménagé évitant essentiellement la dénaturation des acides nucléiques bicaténaires,
– la mise en contact des ARN avec une sonde contenant une séquence de nucléotides complémentaire de l'acide nucléique monocaténaire déterminé, notamment de l'ARN déterminé, dans des conditions propres à permettre l'hybridation de cette sonde avec cet acide nucléique déterminé,

— l'hybridation entre la susdite sonde et la séquence d'acide nucléique monocaténaire déterminée, notamment d'ARN monocaténaire, en présence d'acides nucléiques essentiellement bicaténaires,

— la détection de l'hybride éventuellement formé entre l'acide nucléique déterminé et la séquence complémentaire de la sonde, est caractérisé en ce que:

— on dépose l'ARN sur un filtre approprié pour l'hybridation *in situ* et on lave avec un détergent ionique pour éliminer les protéines.

L'invention s'applique avec un avantage particulier à la détection de mARNs intervenant dans l'expression de gènes particuliers, dès lors que l'on dispose de sondes appropriées.

Un premier élément sur lequel s'appuie l'invention est que la détection d'un ARN messager – ou plus généralement de tout ARN éventuellement présent – dans un échantillon biologique est rendue possible dès que cet ARN peut être "démasqué", ce démasquage impliquant une dissociation au moins partielle des complexes que ces ARN s'avèrent former avec d'autres constituants cellulaires, notamment des protéines. Il n'est alors pas nécessaire de procéder à la purification complète de l'ARN recherché.

Le second élément sur lequel s'appuie le procédé selon l'invention réside dans le traitement ménagé, tel qu'il a été défini plus haut. La non-dénaturation, en tout cas dans des proportions sensibles, des acides nucléiques bicaténaires renforce la sélectivité de l'opération d'hybridation ultérieure de l'ARN recherché avec la sonde marquée. Les ADN bicaténaires ne peuvent en effet pas intervenir avec la réaction d'hybridation.

Le procédé de détection d'ARN selon l'invention est donc particulièrement avantageux en raison de sa simplicité, de sa sensibilité, cette dernière étant encore accrue par la limitation à un minimum de pertes de l'ARN particulier recherché.

Dans l'un de ses modes d'application préférés, le procédé selon l'invention est appliqué à la détection d'un ARN, notamment d'un ARN messager correspondant à une séquence d'ADN affectée par une anomalie génétique.

L'invention est encore applicable à la détection de toute séquence nucléotidique monocaténaire étrangère à la matière biologique étudiée. A ce titre, l'invention peut être appliquée à la détection des produits d'expression de virus, par exemple du virus de l'hépatite B, ou encore de rétrovirus, dont la présence peut attester du développement d'une affection déterminée chez l'hôte dont provient l'échantillon biologique. A titre d'exemple d'affection diagnosticable *in vitro* par le procédé selon l'invention, on mentionnera les lymphadénopathies ou le syndrome de déficience immunitaire acquise, grâce à la détection du rétrovirus qui paraît s'y rattacher (F. Barré-Sinoussi et Coll. Science, 20 mai 1983, 220, 868 - 871).

L'invention trouve une application particulièrement importante dans le domaine du diagnostic précoce *in vitro* de tumeurs malignes ou de leucémies. Elle conduit à des résultats particulièrement significatifs chaque fois qu'un gène oncogène (ci-après désigné souvent sous l'expression "oncogène") lié au type de tumeur en cause a été identifié. Dans un tel cas, la détection reposera sur la réalisation d'une hybridation entre le mARN correspondant, dont la production constitue le premier stade de l'expression du gène correspondant, avec une sonde contenant une séquence nucléotidique correspondant à ce gène.

L'expérience a montré que la méthode de l'invention est applicable au dépistage précoce d'autres tumeurs, notamment de tumeurs solides primaires, même lorsque des oncogènes spécifiques liés à ce type de tumeur n'ont pas encore été isolés. Il a en effet été constaté que le développement au sein d'un organisme d'une tumeur donnée s'accompagne souvent d'une induction de l'expression d'oncogènes, apparemment non directement impliqués dans la tumeur en cause. C'est ce qui est observé dans le cas de carcinomes mammaires humains, qui peuvent être détectés *in vitro* par la mise en oeuvre du procédé selon l'invention avec une sonde hybridable avec le gène Myc, avec un degré de certitude au moins équivalent à celui des résultats fournis par d'autres méthodes utilisées dans ce domaine (si l'on fait exception des coupes anatomo-pathologiques).

Il résulte donc de ce qui précède que l'ARN à détecter n'est pas nécessairement lié de façon directe à la cause biologique même de l'affection qui doit être diagnostiquée. Il suffit qu'il puisse y avoir corrélation entre l'ARN à détecter et l'affection à diagnostiquer.

La matière biologique de choix pour la mise en oeuvre du procédé selon l'invention est constituée par une préparation de cellules sanguines, de préférence des leucocytes. Les observations qui ont été faites apportent un soutien consistant à l'hypothèse qu'une prolifération de cellules tumorales dans l'organismes peut s'accompagner dans de nombreux cas d'une induction suffisante d'une grande variété des oncogènes présents, pour que leur expression soit détectable par le procédé selon l'invention, à l'aide d'une sonde complémentaire de l'un d'entre eux. L'expérience montre en outre que la capacité d'infiltrations de l'organisme par les cellules lymphoblastoides issus de tumeurs solides à caractère très fortement inflammatoire est telle que leur détection est efficacement effectuée sur les leucocytes obtenus à partir d'une simple prise de sang.

L'invention est donc d'une application particulièrement importante pour préciser un diagnostic et un pronostic sur le développement de néoplasies donnant naissance à des métastases (pulmonaires, ganglionnaires...), en ce que le degré d'infiltration de l'organisme, et en particulier du sang par les cellules lymphoblastoides, apparaît comme directement lié à la malignité de la tumeur.

La possibilité de réaliser le diagnostic in vitro sur un échantillon de sang se révèle d'une importance particulière, surtout si l'on tient compte du caractère quelquefois incertain des biopsies souvent effectuées à ce jour pour les besoins du diagnostic in vitro. Outre le désagrément certain que représente les biopsies pour les patients qui les subissent, il peut se faire qu'elles ne soient pas réellement prélevées dans la zone la plus significative de la tumeur, de sorte que le diagnostic peut être faussé. Il va néanmoins sans dire que l'invention peut également être appliquée à une matière biologique consistant en un tissu tumoral, dès lors qu'un diagnostic in vitro plus fin serait requis.

Les possibilités d'application du procédé selon l'invention ne sont pas limitées aux exemples qui viennent d'être indiqués. Il s'applique cependant avec un avantage particulier à la détection de toute affection s'accompagnant de la libération dans la circulation sanguine de produits d'expression de gènes particuliers, que ce soient des affections à caractère cancérigène, par exemple des leucémies, ou des affections rattachées à des anomalies chromosomiques ou à des infections virales.

Conformément à un mode de réalisation préféré du procédé selon l'invention, le traitement ménagé des cellules à traiter comprend une mise en contact des cellules de la matière étudiée, de préférence des leucocytes sanguins, avec un détergent non ionique, tel que ceux connus sous les désignations commerciales ou non, NONIDET 40 (NP 40), BRIJ 35, désoxycholate, etc. et une mise en contact de ceux des constituants cellulaires libérés, susceptibles de contenir les ARN messagers avec une solution d'un sel ionique de haute molarité, par exemple une solution concentrée d'iodure de potassium ou de sodium pour à la fois dissoudre et dissocier les complexes que forment les ARN avec des protéines ou autres constituants.

Les extraits cellulaires peuvent alors être soumis à la procédure de détection de l'ARN complémentaire de celui de la sonde choisie. On peut à cet égard avoir recours à toute technique connue. Il est avantageux d'utiliser les techniques d'hybridation in situ de nitrocellulose. A cet effet, un petit volume de la solution de l'extrait cellulaire et du sel ionique est déposé sur le filtre de cellulose. Ce dernier est ensuite lavé avec les solution permettant d'éliminer les excès du sel ionique, des protéines et de la, sonde non fixée. Il est avantageux de procéder à ces lavages avec de l'eau, des solutions d'un détergent ionique tel que le dodécyl sulfate de sodium, et d'éthanol. Ce dernier contribue également à fixer l'ARN précipité sur le filtre et facilite la détection ultérieure, par mise en contact du filtre avec une sonde marquée.

Il va de soi que l'on peut utiliser toute technique classique pour réaliser le marquage de la sonde. Par exemple, celle-ci est marquée radioactivement ou couplée (ou couplable) à une enzyme révélable par l'action qu'elle est susceptible d'exercer à l'égard d'un substrat déterminé, ou à une molécule fluorescente.

Il est avantageux de procéder à une acétylation ou analogue du filtré, avant la réalisation des mesures aboutissant à la détection, dans le but d'empêcher tout bruit de fond, tels que ceux qui seraient produits par les interactions de la sonde acide nucléique avec les charges du filtre.

L'invention concerne encore des nécessaires pour réaliser la détection in vitro d'un acide nucléique normalement monocaténaire déterminé, notamment d'un ARN déterminé dans une matière biologique, comprenant un ensemble de réactifs-parmi lesquels:

- un ou plusieurs détergents non ioniques permettant, dans des conditions de dilution appropriées, de dissoudre les membranes des cellules contenues dans la matière biologique à traiter;

- les constituants et tampons nécessaires à la réalisation des solutions des susdits détergents non ioniques aux concentrations permettant la libération d'une partie au moins des constituants cellulaires internes, y compris des ARNs messagers;

- des sels ioniques permettant le traitement ménagé des cellules de la susdite matière pour réaliser, à une molarité de 5 à 13 M, la dissociation des complexes protéines-ARNs contenus dans la matière traitée et pour démasquer les ARNs;

- les constituants et tampons nécessaires à la réalisation des solutions des susdits sels ioniques aux susdites concentrations permettant le traitement ménagé des cellules de la susdite matière, pour dissocier les complexes protéines-ARN et pour démasquer les ARNs messagers;

- une ou plusieurs sondes marquées contenant une séquence de nucléotides complémentaire de l'acide nucléique normalement monocaténaire déterminé et les moyens permettant la réalisation de l'hybridation ultérieure in situ de l'ARN caractéristique de l'anomalie génétique recherchée avec une ou plusieurs des susdites sondes marquées, caractérisé en ce qu'il comprend également;

- un détergent ionique pour éliminer les protéines par lavage,

- les constituants et tampons nécessaires à la réalisation de la soiution du susdit détergent ionique.

Avantageusement, ce nécessaire comporte encore les moyens pour réaliser une hybridation in situ entre le ou les sondes et l'ARN complémentaire éventuellement présent dans le milieu biologique étudié. Le nécessaire comprend alors un filtre ou analogue sur lequel peuvent être déposés les ARN, et un réactif de fixation de l'ARN messager sur le filtre, tel que l'éthanol, les réactifs pour l'élimination par lavage des protéines, lipides et autres constituants cellulaires résiduels.

Ce nécessaire comprend avantageusement encore un réactif tel que l'anhydride acétique dans la triéthanolamine, apte à prévenir la fixation de la sonde sur le filtre, par l'intermédiaire de liaisons autres que celles résultant de l'hybridation.

Des solutions avantageuses des divers réactifs susceptibles d'être utilisés efficacement dans la mise en oeuvre du procédé selon l'invention présentent notamment les domaines de concentrations suivants:

- solution aqueuse de détergent non ionique: de 0,2 % à 1 % en volume de NP 40 ou 0,2 à 1 % en volume de désoxycholate, ou mélange des deux solutions à raison de 20 à 80 parties de NP 40 et de 80 à 20 parties de désoxycholate, ou de préférence équiparties,
- solution aqueuse d'iodure de sodium ou de potassium 5 à 13 M,
- solution du détergent ionique, tel que le dodécylsulfate de sodium: 0,2 à 2 % en volume,
- solution aqueuse d'éthanol: 50 à 100 % en volume,
- solution d'anhydride acétique dans la triéthanolamine: 0,05 à 0,2 % molaire en triéthanolamine et 0,1 à 0,5 % en volume d'acide acétique.

Dans ce qui suit, on indique à titre d'exemple non limitatif une procédure avantageuse pour réaliser un essai type de détection de l'expression d'un oncogène dans un échantillon biologique provenant d'un patient déterminé.

Un échantillon de sang est soumis à un traitement visant à lyser les globules rouges, par exemple par mise en contact avec une solution de chlorure d'ammonium. On centrifuge l'échantillon à basse vitesse, par exemple à 1000 rpm pendant 5 minutes. On récupère le culot de centrifugation contenant les leucocytes. Les leucocytes prélevés sont lavés deux fois dans un tampon phosphate (PBS) et dissociés dans un mélange de deux détergents à 0,5 % (NP 40 et désoxycholate). Après une incubation à 0°C pendant une durée de 5 à 15 minutes suivant la nature du tissu, les cellules sont traitées à la température ordinaire avec l'iodure de sodium (NaI, 6,1 M). L'extrait cellulaire obtenu peut être dilué dans une solution saturée de NaI (12,2 M) et déposé dans un petit volume (quelques microlitres) sur un filtre de nitrocellulose (Schleischer et Schull). Les filtres sont lavés 5 minutes dans l'eau, puis dans une solution de dodécyl sulfate de sodium à 1 %. Ensuite trois lavages de 5 minutes sont effectués dans l'éthanol à 70 % pour éliminer l'iodure de sodium. Enfin un lavage de 10 minutes dans 20 ml d'une solution contenant 0,25 ml d'anhydride acétique dans 100 ml de triéthanolamine éliminera le bruit de fond du test. Les filtres sont séchés et peuvent être stockés dans un dessicateur.

Le filtre est ensuite traité et hybridé pendant une nuit avec un ADN sonde marqué (contenant par exemple une séquence hybridable avec l'un des oncogènes Myc, Myb, Erb, etc.), par exemple comme décrit par Wahl et al. (1979)... Les séquences homologues (Myc, Myb, Erb) hybridées peuvent, par exemple, être détectées par autoradiographie de la sonde radioactive ou avec un antisérum antibiotine reconnaissant l'ADN sonde, lorsque l'on a recours à une sonde à laquelle a été couplée (ou peut être couplée) la biotine. L'intensité de la tache ("spot") permet d'apprécier de façon précise le degré d'expression de l'oncogène par cellule, par gramme de tissu ou par millilitre de sang. Le filtre contenant toujours un standard interne peut être rehybridé avec une autre sonde marquée.

Ce test a été mis en oeuvre dans le dépistage du carcinome mammaire humain sur des biopsies ou prélèvements de leucocytes provenant de 25 patients humains. Il a conduit à 12 réponses positives. Les résultats présentent une bonne corrélation avec ceux qui ont été obtenus par l'examen de coupes anatomo-pathologiques.

Comme il va de soi et comme il résulte d'ailleurs déjà de ce qui précède, l'invention ne se limite nullement à ceux de ses modes d'application et de réalisation qui ont été plus spécialement envisagés: elle en embrasse au contraire toutes les variantes.

**Revendications**

1. Procédé de détection in vitro d'un acide nucléique normalement monocaténaire, notamment d'un ARN déterminé, dans une matière biologique le contenant et contenant des cellules, comprenant les étapes suivantes:

- le traitement de la matière biologique par la mise en contact de ladite matière avec un détergent non ionique pour solubiliser les membranes des cellules contenues dans la matière biologique et libérer une partie des constituants cellulaires internes y inclus les ADN, ARN, et plus particulièrement les ARN messagers,
- la dissociation, avant l'hybridation des complexes protéine-ARN monocaténaire et le démasquage de l'ARN monocaténaire à l'aide d'une solution d'un sel ionique, de molarité de 5 à 13 M, ce traitement ménagé évitant essentiellement la dénaturation des acides nucléiques bicaténaires,
- la mise en contact des ARN avec une sonde contenant une séquence de nucléotides complémentaire de l'acide nucléique monocaténaire déterminé, notamment de l'ARN déterminé, dans des conditions propres à permettre l'hybridation de cette sonde avec cet acide nucléique déterminé,
- l'hybridation entre la susdite sonde et la séquence d'acide nucléique monocaténaire déterminée, notamment d'ARN monocaténaire, en présence d'acides nucléiques essentiellement bicaténaires. caractérisé en ce que:
- la détection de l'hybride éventuellement formé entre l'acide nucléique déterminé et la séquence complémentaire de la sonde,
- on dépose l'ARN sur un filtre approprié pour l'hybridation in situ et on lave avec un détergent ionique pour éliminer les protéines.

2. Procédé selon la revendication 1, caractérisé en ce que l'on dépose l'ARN sur un filtre approprié pour l'hybridation in situ et on lave avec un détergent ionique pour éliminer les protéines, avant de procéder à une acétylation ou analogue du filtre dans le but d'empêcher tout bruit de fond.

3. Procédé selon la revendication 1 ou 2, caractérisé en ce que après le dépôt de l'ARN sur le filtre et le lavage avec un détergent ionique, on lave avec de l'anhydride acétique dans de la triéthanolamine pour éliminer le bruit de fond.

4. Procédé selon les revendications 1 à 3, caractérisé en ce que le détergent ionique pour éliminer les protéines par lavage est du dodécylsulfate de sodium.

5. Procédé selon l'une quelconque des revendications 1 à 4, caractérisé par le fait que l'ARN à détecter consiste en un ARN messager intervenant dans l'expression d'un gène déterminé.

6. Procédé selon l'une quelconque des revendications 1 à 4, caractérisé en ce que l'ARN à détecter est caractéristique d'une anomalie génétique.

7. Procédé selon l'une quelconque des revendications 1 à 4, caractérisé en ce que l'ARN de l'acide nucléique normalement monocaténaire à détecter est étranger au patrimoine génétique des cellules et normalement est constitué par un ADN viral ou par un ARN viral.

8. Procédé selon l'une quelconque des revendications 1 à 4, caractérisé en ce que l'ARN déterminé est celui qui résulte de la transcription d'un oncogène.

9. Procédé selon l'une quelconque des revendications 1 à 8, caractérisé par le fait que l'échantillon biologique est formé de constituants cellulaires du sang.

10. Procédé selon la revendication 9, caractérisé en ce que l'échantillon biologique est à base de leucocytes.

11. Application du procédé selon l'une quelconque des revendications 1 à 10 à la détection in vitro d'une anomalie génétique à caractère pathologique par mise en contact des susdits constituants cellulaires avec une sonde contenant une séquence nucléotidique hybridable avec un ARN messager correspondant au gène affecté par l'anomalie génétique.

12. Application du procédé selon l'une quelconque des revendications 1 à 10 à la détection in vitro de cellules blastoïdes ou de métastases de tumeurs malignes, caractérisée par la mise en contact des susdits constituants cellulaires avec une sonde complémentaire d'un gène oncogène.

13. Nécessaire pour réaliser la détection in vitro d'un acide nucléique normalement monocaténaire déterminé, notamment d'un ARN déterminé dans une matière biologique, comprenant un ensemble de réactifs parmi lesquels:

- un ou plusieurs détergents non ioniques permettant, dans des conditions de dilution appropriées, de dissoudre les membranes des cellules contenues dans la matière biologique à traiter;
- les constituants et tampons nécessaires à la réalisation des solutions des susdits détergents non ioniques aux concentrations permettant la libération d'une partie au moins des constituants cellulaires internes, y compris des ARNs messagers;
- des sels ioniques permettant le traitement ménagé des cellules de la susdite matière pour réaliser, à une molarité de 5 à 13 M, la dissociation des complexes protéines-ARNs contenus dans la matière traitée et pour démasquer les ARNs;
- les constituants et tampons nécessaires à la réalisation des solutions des susdits sels ioniques aux susdites concentrations permettant le traitement ménagé des cellules de la susdite matière, pour dissocier les complexes protéines-ARN et pour démasquer les ARNs messagers;
- une ou plusieurs sondes marquées contenant une séquence de nucléotides complémentaire de l'acide nucléique normalement monocaténaire déterminé et les moyens permettant la réalisation de l'hybridation ultérieure in situ de l'ARN caractéristique de l'anomalie génétique recherchée avec une ou plusieurs des susdites sondes marquées, caractérisé en ce qu'il comprend également:
- un détergent ionique pour éliminer les protéines par lavage,
- les constituants et tampons nécessaires à la réalisation de la solution du susdit détergent ionique.

14. Nécessaire selon la revendication 13, caractérisé en ce que la matière biologique est formée par les constituants cellulaires du sang.

15. Nécessaire selon la revendication 14, caractérisé en ce que la matière biologique est à base de leucocytes.

16. Nécessaire selon l'une quelconque des revendications 13 à 15, caractérisé en ce que les sels ioniques sont des sels de iodure de sodium ou des sels de iodure de potassium.

17. Nécessaire selon l'une quelconque des revendications 13 à 16, caractérisé en ce que les moyens pour réaliser l'hybridation in situ comprennent un filtre sur lequel peuvent être déposés les acides nucléiques, un réactif de fixation des acides nucléiques monocaténaires sur le filtre et un détergent ionique pour l'élimination par lavage des protéines, lipides et autres constituants cellulaires résiduels.

18. Nécessaire selon la revendication 17, caractérisé en ce que le réactif de fixation est l'éthanol et le susdit détergent ionique pour l'élimination des protéines est le dodécylsulfate de sodium.

19. Nécessaire selon l'une quelconque des revendications 17 ou 18 comprenant comme réactif l'anhydride acétique dans la triéthanolamine, apte à prévenir la fixation de la sonde sur le filtre, par l'intermédiaire de liaisons autres que celles résultant de l'hybridation.

**Claims**

1. Process for the in vitro detection of a normally monocatenary nucleic acid, particularly of a determined RNA, in a biological medium containing it and containing cells, comprising the following steps:

- treating the biological material by contacting said material with a non ionic detergent to solubilize the membranes of the cells contained in said biological material and release a part of the internal cellular constituents included in DNA, RNA and more particularly RNA messengers;
- dissociating, before hybridization, the monocatenary protein-RNA complexes, and unmasking the monocatenary RNA by means of a solution of an ionic salt, having a molarity from 5 M to 13 M, this mild treatment avoiding substantially the denaturation of bicatenary nucleic acids;
- contacting RNAs with a probe containing a complementary nucleotide sequence of the determined monocatenary nucleic acid, particularly of the determined RNA, under conditions enabling to allow hybridization of this probe with this determined nucleic acid;
- hybridization between the abovesaid probe and the determined monocatenary nucleic acid sequence, particularly the monocatenary RNA, in the presence of substantially bicatenary nucleic acids;
- detecting the hybrid possibly formed between the determined nucleic acid and the complementary sequence of the probe;

characterized in that RNA is deposited on an appropriate filter for the in situ hybridization and eliminate proteins.

2. Process according to claim 1, characterized by the fact that RNA is deposited on an appropriate filter for in situ hybridization and washing is carried out with an ionic detergent to eliminate the proteins, before carrying out an acetylation or analogous step of the filter in order to avoid any background noise.

3. Process according to claim 1 or 2, characterized by the fact that after the deposit of RNA onto the filter and the washing with an ionic detergent, washing is carried out with acetic anhydride in triethanolamine to avoid the background noise.

4. Process according to claims 1 to 3, characterized by the fact that the ionic detergent to eliminate the proteins by washing is sodium dodecylsulfate.

5. Process according to anyone of claims 1 to 4, characterized by the fact that the RNA to be detected consists in a messenger RNA occuring in the expression of a determined gene.

6. Process according to anyone of claims 1 to 4, characterized by the fact that the RNA to be detected is characteristic of a genetic abnormality.

7. Process according to anyone of claims 1 to 4, characterized by the fact that the RNA of the normally monocatenary nucleic acid to be detected is foreign to the genetic patrimony of the cells and is normally constituted by a viral DNA or by a viral RNA.

8. Process according to anyone of claims 1 to 4, characterized by the fact that the determined RNA is the one which results from the transcription of an oncogene.

9. Process according to anyone of claims 1 to 8, characterized by the fact that the biological sample is formed of blood cellular constituents.

10. Process according to claim 9, characterized by the fact that the biological sample is based on leucocytes.

11. Use of the process according to anyone of claims 1 to 10, for the in vitro detection of a genetic abnormality of pathological character by contacting the abovesaid cellular constituents with a probe containing a nucleotidic sequence which can be hybridized with a messenger RNA corresponding to the gene affected by the genetic abnormality.

12. Use of the process according to anyone of claims 1 to 10, for the in vitro detection of blastoids cells or metastases of malignant tumors characterized by contacting the abovesaid cellular constituents with a probe complementary of an oncogenic gene.

13. Kit to carry out the in vitro detection of a

13

determined normally monocatenary nucleic acid, particularly a determined RNA in a biological medium comprising a set of reagents among which:

- one or several non ionic detergents enabling under appropriate dilution conditions, to dissolve the membranes of cells contained in the biological material to be treated;
- components and buffers necessary for preparing the solutions of said non ionic detergents at concentrations enabling the release of at least a part of the cellular internal constituents, included messenger RNAs;
- ionic salts enabling the mild treatment of cells of the abovesaid material to carry out at a molarity of 5 to 13 M, the dissociation of protein-RNA complexes contained in the material to be treated and to unmask the RNAs;
- components and buffers necessary for preparing the solutions of abovesaid ionic salts at the abovesaid concentrations enabling the mild treatment of the cells of abovesaid material for dissociating the RNA-protein complexes and for unmasking the messenger RNAs;
- one or several labeled probes containing a complementary nucleotide sequence of the determined normally monocatenary nucleic acid and means enabling to carry out the further in situ hybridization of the RNA characteristics of the salt genetic abnormality with one or several of the abovesaid labeled probes, characterized in that it also comprises:
- an ionic detergent to eliminate the proteins by washing;
- the constituents and buffers necessary for preparing the solution of the abovesaid ionic detergent.

14. Kit according to claim 13, characterized by the fact that the biological material is formed by the cellular constituents of blood.

15. Kit according to claim 14, characterized by the fact that the biological material is based on leucocytes.

16. Kit according to anyone of claims 13 to 15, characterized by the fact that the ionic salts are sodium iodide salts or potassium iodide salts.

17. Kit according to anyone claims 13 to 16, characterized by the fact that means to carry out in the in situ hybridization comprise a filter on which the nucleic acids can be deposited, a fixation reagent of the monocatenary nucleic acids on the filter and an ionic detergent to eliminate proteins by washing, lipids and other residual cellular constituents.

18. Kit according to claim 17, characterized by the fact that the fixation reagent is ethanol and the abovesaid ionic detergent to eliminate the proteins is sodium dodecylsulfate.

19. Kit according to anyone claims 17 or 18,

14

comprising as reagent acetic anhydride in triethanolamine, able to prevent the fixation of the probe on the filter, by the intermediary of bindings other than the ones resulting from hybridization.

## Patentansprüche

1. Verfahren zum in vitro Nachweis einer normalerweise einkettigen Nukleinsäure, insbesondere einer bestimmten RNS, in einem sie und Zellen enthaltenden biologischen Material, mit den folgenden Verfahrenschritten:

- die Behandlung des biologischen Materials durch die Herstellung eines Kontakts des Materials mit einem nicht ionischen Detergens, um die Membranen der in dem biologischen Material enthaltenen Zellen lösbar zu machen und einen Teil der internen Zellenbestandteile freizusetzen, darunter DNS, RNS und insbesondere die Messenger-Ribonkleinsäuren,
- die Dissoziierung vor der Hybridisierung der Komplexe von Protein und einkettiger RNS und Demaskierung der einkettigen RNS mit Hilfe einer Lösung eines ionischen Salzes der Molarität 5 - 13 M, wobei diese schonende Behandlung im wesentlichen die Denaturierung der doppelkettigen Nukleinsäuren vermeidet,
- das Inkontaktbringen der RNS mit einer eine komplementäre Nukleotidsequenz der bestimmten einkettigen Nukleinsäure, insbesondere der bestimmten RNS, enthaltenden Sonde unter Bedingungen, die geeignet sind, die Hybridisierung dieser Sonde mit dieser bestimmten Nukleinsäure zu erlauben,
- die Hybridisierung zwischen der genannten Sonde und der bestimmten Sequenz der einkettigen Nukleinsäure, insbesondere der einkettigen RNS, in Anwesenheit von im wesentlichen doppelkettigen Nukleinsäuren,
- die Feststellung des gegebenenfalls gebildeten Hybrids zwischen der bestimmten Nukleinsäure und der komplementären Sequenz der Sonde,
- dadurch gekennzeichnet, daß man die RNS auf ein für die Hybridisierung in situ geeignetes Filter aufbringt und mit einem ionischen Detergenz wäscht, um die Proteine zu eliminieren.

2. Verfahren nach Anspruch 1, dadurch gekennzeichnet, daß man die RNS auf ein für die Hybridisierung in situ geeignetes Filter bringt und mit einem ionischen Detergenz wäscht, um die Proteine zu eliminieren, bevor man eine Acetylierung oder dergleichen des Filters vornimmt, um Hintergrundstörungen zu vermeiden.

3. Verfahren nach Anspruch 1 oder 2, dadurch gekennzeichnet, daß man nach der Aufbringung der RNS auf das Filter und dem Waschen mit einem ionischen Detergens mit Essigsäureanhydrid in Triethanolamin wäscht, um Hintergrundstörungen zu eliminieren.

4. Verfahren nach den Ansprüchen 1 - 3, dadurch gekennzeichnet, daß das ionische Detergenz zum Eliminieren der Proteine durch Waschen Natriumdodecylsulfat ist.

5. Verfahren nach einem der Ansprüche 1 - 4, dadurch gekennzeichnet, daß die nachzuweisende RNS aus einer Messenger-RNS besteht, die bei der Expression eines bestimmten Gens interveniert.

6. Verfahren nach einem der Ansprüche 1 - 4, dadurch gekennzeichnet, daß die nachzuweisende RNS charakteristisch für eine genetische Anomalie ist.

7. Verfahren nach einem der Ansprüche 1 - 4, dadurch gekennzeichnet, daß die nachzuweisende RNS der normalerweise einkettigen Nukleinsäure dem genetischen Erbgut der Zellen fremd ist und normalerweise von einer viralen DNS oder einer viralen RNS gebildet wird.

8. Verfahren nach einem der Ansprüche 1 - 4, dadurch gekennzeichnet, daß die bestimmte RNS diejenige ist, die aus der Transkription eines Onkogens resultiert.

9. Verfahren nach einem der Ansprüche 1 - 8, dadurch gekennzeichnet, daß das biologische Muster von Zellenbestandteilen des Blutes gebildet wird.

10. Verfahren nach einem Anspruch 9, dadurch gekennzeichnet, daß das biologische Muster eines auf Basis von Leukozyten ist.

11. Anwendung des Verfahrens nach einem der Ansprüche 1 - 10 zum in vitro Nachweis einer genetischen Anomalie mit pathologischem Charakter durch das Inkontaktbringen der oben genannten zellulären Bestandteile mit einer Sonde, die eine Nukleotidsequenz enthält, die mit einer dem durch die genetische Anomalie betroffenen Gen entsprechenden Messenger-RNS hybridisierbar ist.

12. Anwendung des Verfahrens nach einem der Ansprüche 1 - 10 auf den in vitro Nachweis von Blastoidzellen oder von Metastasen bösartiger Tumore, gekennzeichnet durch das Inkontaktbringen der oben genannten Zellenbestandteile mit einer Komplementärsonde eines onkogenen Gens.

13. Material für die Durchführung des in vitro Nachweises einer bestimmten normalerweise einkettigen Nukleinsäure, insbesondere einer bestimmten RNS, in einem biologischen Material, enthaltend eine Anzahl von Reagentien, zu denen gehören:

- ein oder mehrere nicht-ionische Detergentien, die es unter geeigneten Verdünnungsbedingungen ermöglichen, die Membranen der in dem zu behandelnden biologischen Material enthaltenen Zellen auflösen;

- die Bestandteile und Puffer, die zur Realisierung der Lösungen der oben genannten nichtionischen Detergentien bei Konzentrationen nötig sind, die die Freisetzung mindestens eines Teils der internen Zellenbestandteile ermöglichen, unter ihnen die Messenger-Ribonkleinsäuren;

- ionische Salze, die die schonende Behandlung der Zellen des oben genannten Materials ermöglichen, u. bei einer Molarität von 5 - 13 M die Dissozierung der Komplexe Protein-RNSn, die in dem behandelten Material enthalten sind, zu realisieren und die RNSn zu demaskieren,

- die Bestandteile und Puffer, die zur Realisierung der Lösungen der oben genannten ionischen Salze bei den genannten Konzentrationen erforderlich sind, die die schonende Behandlung der Zellen des genannten Materials ermöglichen, um die Komplexe Protein-RNS zu dissozieren und die Messenger-Ribonkleinsäurenzu demaskieren,

- eine oder mehrere markierte Sonden, die eine komplementäre Nukleotidsequenz der bestimmten normalerweise einkettigen Nukleinsäure enthalten und die Mittel, die die Realisierung der folgenden Hybridisierung in situ der für die gesuchte genetische Anomalie charakteristischen RNS mit einer oder mehreren der oben genannten markierten Sonden ermöglichen, dadurch gekennzeichnet, daß es ebenfalls umfaßt:

- ein ionisches Detergenz zum Eliminieren der Proteine durch Waschen,

- die Bestandteile und Puffer, die zur Realisierung der Lösung des oben genannten ionischen Detergenz erforderlich sind.

14. Material nach Anspruch 13 dadurch gekennzeichnet, daß das biologische Material durch die Zellenbestandteile des Blutes gebildet wird.

15. Material nach Anspruch 14 dadurch gekennzeichnet, daß das biologische Material solches auf Basis von Leukozyten ist.

16. Material nach einem der Ansprüche 13 - 15 dadurch gekennzeichnet, daß die ionischen Salze Salze von Natriumjodid oder Salze von Kaliumjodid sind.

17. Material nach einem der Ansprüche 13 - 16, dadurch gekennzeichnet, daß die Mittel zur Realisierung der Hybridisierung in situ ein Filter, auf dem die Nukleinsäuren abgelegt werden können, ein Reagenz zur Fixierung der einkettigen Nukleinsäuren auf dem Filter und ein ionisches Detergens zur Eleminierung der Proteine, Lipide und anderer Restzellenbestandteile durch Waschen umfassen.

18. Material nach Anspruch 17 dadurch gekennzeichnet, daß das Fixierungsreagens Ethanol und das genannte ionische Detergens für die Eliminierung der Proteine Natriumdodecylsulfat ist.

19. Material nach einem der Ansprüche 17 oder 18, enthaltend als Reagenz Essigsäureanhydrid in Triäthanolamin, geeignet zur Verhinderung der Fixierung der Sonde auf dem Filter, über andere Bindungen als die, die sich aus der Hybridisierung ergeben.